# FASCICULE DE BREVET EUROPEEN

(11) **EP 2 271 291 B1**
(45) Date de publication et mention de la délivrance du brevet: **27.02.2013**
(21) Numéro de dépôt: 09722617.9
(22) Date de dépôt: 02.02.2009
(51) Int. Cl.: A61F 5/01

(54) **ORTHESE DE POIGNET, DESTINEE AU TRAITEMENT DU SYNDROME DU CANAL CARPIEN**
HANDGELENKORTHOSE ZUR BEHANDLUNG VON KARPALTUNNELSYNDROM
WRIST ORTHOSIS FOR TREATING CARPAL TUNNEL SYNDROME

(30) Priorité: 21.03.2008 FR 0801568
(43) Date de publication de la demande: 12.01.2011
(73) Titulaire: Gibaud, 42000 Saint Etienne (FR)
(72) Inventeur: ANGLADA, Gérard, F-42000 Saint Etienne (FR)
(74) Mandataire: Delorme, Nicolas
(86) Numéro de dépôt international: PCT/FR2009/000114
(87) Numéro de publication internationale: WO 2009/115661

(56) Documents cités:
- WO-A-95/25489
- GB-A- 2 184 659
- US-A- 5 358 471
- US-A1- 2007 276 305
- US-B1- 6 293 918

## Description

La présente invention concerne une orthèse de poignet, destinée au traitement du syndrome du canal carpien (inflammation des muscles fléchisseurs).

Les pathologies dans la région du canal carpien peuvent nécessiter une intervention chirurgicale. Or, on sait que cette intervention peut être évitée dans 50% des cas si, dès l'apparition des premiers symptômes, on empêche la flexion du poignet, notamment en phase nocturne.

Ainsi, on connaît déjà des orthèses destinées à empêcher la flexion du poignet de l'utilisateur. Initialement, lorsque les symptômes sont les plus importants, il est préconisé de placer le poignet dans une position de traitement correspondant à un certain degré d'extension et d'empêcher la flexion du poignet à partir de cette position. Par la suite, en fonction de la récupération de l'utilisateur, et de façon progressive, la position de traitement peut correspondre à un degré d'extension moindre, voire à un certain degré de flexion, la flexion à partir de cette position étant toujours bloquée.

On connaît déjà des orthèses de ce type réalisées sur mesure par un orthésiste. Elles consistent en une pièce rigide maintenue autour du poignet et de l'avant-bras par des sangles, cette pièce rigide définissant une position de traitement déterminée. En fonction de l'évolution des symptômes de l'utilisateur, l'orthésiste modifie la position de traitement. A cet effet, ce type d'orthèse est réalisé en un matériau qui, lorsqu'il dépasse une certaine température, devient malléable, et qui redevient rigide après refroidissement. Par exemple, l'orthèse est réalisée en résine et l'orthésiste l'immerge dans l'eau chaude pour pouvoir ensuite la modeler et l'adapter au stade de guérison de l'utilisateur en fixant le degré d'extension ou de flexion approprié.

Ces orthèses requièrent donc l'intervention d'un orthésiste qualifié, et ne peuvent être adaptées par l'utilisateur lui-même. De plus, les opérations de changement de la position de traitement, c'est-à-dire de l'angle d'extension ou de flexion, sont relativement longues. Il s'agit donc, pour éviter que le traitement ne soit interrompu pendant quelques jours, d'apporter l'orthèse chez le spécialiste à un moment où il est disponible pour effectuer le remodelage, et de venir la récupérer rapidement après qu'il a procédé à ce remodelage. Bien entendu, ce moment doit également correspondre au moment où l'évolution des symptômes requiert une modification de la position de traitement définie par l'orthèse. Tout cela est particulièrement contraignant.

On connaît par ailleurs, notamment des documents GB 2 184 659 et US 6 293 918, des orthèses comprenant une partie proximale destinée à enserrer l'avant-bras et une partie distale destinée à enserrer la main, dans lesquelles l'angle entre les parties proximale et distale peut être modifié, par pivotement relatif autour d'un axe sensiblement confondu avec l'axe d'articulation en flexion / extension du poignet de l'utilisateur.

Toutefois, ces orthèses connues ne donnent pas entièrement satisfaction, notamment pour des questions de facilité de mise en oeuvre et de qualité du maintien.

La présente invention vise à remédier aux inconvénients mentionnés ci-dessus.

A cet effet, l'invention concerne une orthèse de poignet selon la revendication 1.

Grâce à l'invention, il devient très facile et très rapide de modifier la position de traitement par un simple actionnement des moyens de pivotement puis un blocage de la partie distale par rapport à la partie proximale dans la position angulaire souhaitée. La position de traitement appropriée peut être définie par un médecin, en fonction de l'évolution des douleurs ressenties par l'utilisateur, tandis que l'opération de modification de l'angle relatif peut être réalisée par l'utilisateur lui-même.

Par conséquent, l'évolution de la forme de l'orthèse peut suivre très précisément la récupération progressive de l'utilisateur. L'invention fournit donc une orthèse particulièrement bien adaptée à tous les stades de guérison de la pathologie de l'utilisateur et, in fine, permettant un traitement amélioré du syndrome du canal carpien.

De plus, le fait que les parties rigides de l'orthèse soient placées respectivement « sur » le bras et « sous » la main est très avantageux puisque, grâce à cette disposition, on obtient un effet bras de levier qui garantit un excellent maintien de l'orthèse.

Ce maintien est obtenu avec une pression moins importante que si les deux parties rigides étaient situées du même côté, c'est-à-dire uniquement du côté antérieur - ou postérieur - de l'avant-bras et de la main. En effet, avec la disposition selon l'invention, il n'est pas nécessaire de serrer les sangles autant que dans l'art antérieur pour garantir ce maintien. Il s'ensuit une diminution de la compression, donc plus de confort et des risques vasculaires réduits pour l'utilisateur.

En outre, l'orthèse selon l'invention est particulièrement adaptée au traitement du syndrome du canal carpien du fait de l'absence de toute partie rigide sous le canal carpien. Le confort de port s'en trouve également accru.

L'expression « sensiblement rigide » est employée pour le brassard par opposition à la sangle qui est totalement souple et déformable. Mais cela n'exclut pas que le brassard puisse avoir une certaine capacité à être déformé élastiquement selon une faible amplitude. La présence d'un brassard sensiblement rigide rend l'orthèse plus simple à mettre et à régler pour l'utilisateur que si l'orthèse était fixée autour de l'avant-bras uniquement par un jeu de sangles.

L'orthèse peut en outre comporter des moyens de limitation de l'amplitude de pivotement de la partie distale par rapport à la partie proximale dans une plage angulaire s'étendant de part et d'autre de la position neutre. La position neutre correspond à une position dans laquelle le poignet du porteur de l'orthèse n'est ni en flexion ni en extension. Ces moyens de limitation constituent un dispositif de sécurité, car ils permettent d'empêcher que l'orthèse ne forme un angle trop important qui pourrait être préjudiciable à la guérison de l'utilisateur.

Selon une réalisation possible, l'orthèse comprend des moyens de réglage de l'angle entre la partie distale et la partie proximale selon des valeurs d'angle discrètes, par exemple de 5° en 5°.

On décrit à présent, à titre d'exemple non limitatif, un mode de réalisation possible de l'invention, en référence aux figures annexées :
La figure 1 est une vue en perspective avant d'une orthèse selon l'invention ;
La figure 2 est une vue en perspective arrière de l'orthèse;

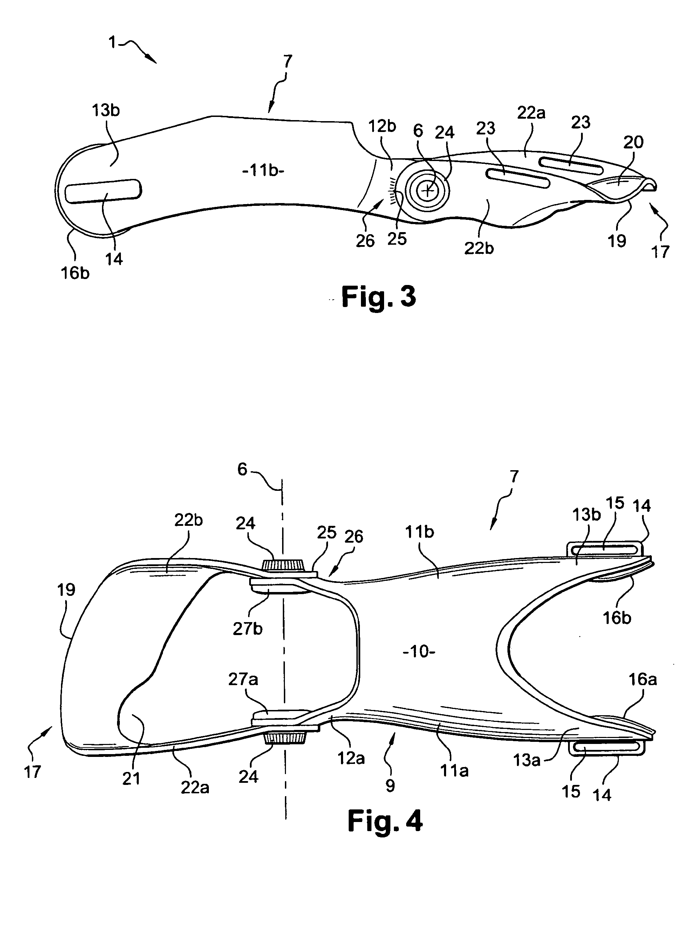

La figure 3 est une vue latérale de l'orthèse;
La figure 4 est une vue de dessus de l'orthèse;
La figure 5 est une vue arrière de l'orthèse;
La figure 6 est une vue de dessous de l'orthèse portée par un utilisateur ; et
Les figures 7a à 7e sont des vues latérales de l'orthèse portée par un utilisateur, avec des angles différents entre la partie distale et la partie proximale de l'orthèse.

Comme illustré sur les figures, une orthèse 1 selon l'invention comprend une partie proximale 2 destinée à enserrer l'avant-bras 3 d'un utilisateur et une partie distale 4 destinée à enserrer sa main 5.

On définit la direction longitudinale D1 comme la direction générale dans laquelle s'étend l'avant-bras (lorsque l'orthèse 1 est portée), la direction transversale D2 comme la direction de l'articulation du poignet en flexion /extension et la direction verticale D3 comme la direction de l'articulation du poignet en adduction / abduction.

Les termes « proximal » et « distal » sont employés en référence à la direction D1 et le terme « latéral » en référence à la direction D2.

Les parties proximale 2 et distale 4 forment deux pièces distinctes qui peuvent pivoter l'une par rapport à l'autre autour d'un axe de pivotement 6 transversal qui, lorsque l'orthèse 1 est portée par un utilisateur, est sensiblement confondu avec l'axe d'articulation en flexion / extension du poignet de l'utilisateur.

On décrit tout d'abord la partie proximale 2.

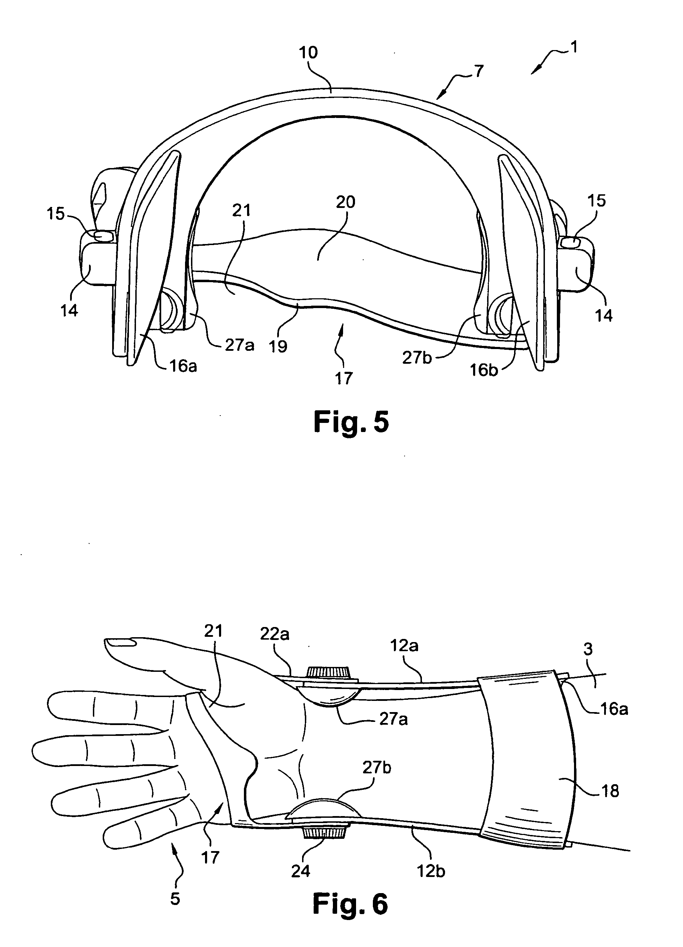

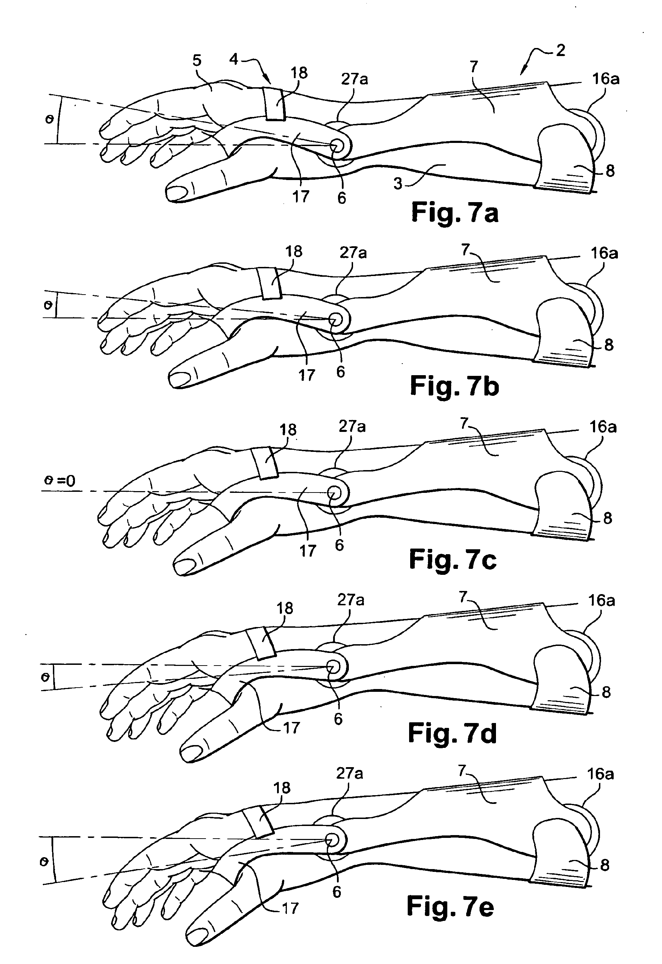

La partie proximale 2 comporte un brassard 7 sensiblement rigide, par exemple réalisé en matière plastique ou en résine, et une sangle 8 souple reliée au brassard 7 (voir figures 6 et 7a à 7e).

Dans la réalisation représentée, le brassard 7 comporte :
- une partie principale 9 en forme de gouttière qui, lors du port de l'orthèse 1 par un utilisateur, vient coiffer l'avant-bras, ladite partie principale 9 comprenant une portion centrale 10 placée contre la face postérieure de l'avant-bras et deux portions latérales 11 a, 11 b disposées de part et d'autre de l'avant-bras ;
- deux pattes latérales distales 12a, 12b s'étendant sensiblement longitudinalement dans le sens distal chacune depuis une portion latérale 11 a, 11 b de la partie principale 9, de part et d'autre de l'avant-bras, l'axe de pivotement 6 étant situé sensiblement à la partie extrême distale desdites pattes latérales distales 12a, 12b.

Le brassard 7 comporte en outre deux pattes latérales proximales 13a, 13b s'étendant sensiblement longitudinalement dans le sens proximal chacune depuis une portion latérale 11a, 11 b de la partie principale 9, de part et d'autre de l'avant-bras, lesdites pattes latérales proximales 13a, 13b formant une fourche pouvant être légèrement déformée élastiquement. Ainsi, en choisissant un écartement adapté entre les pattes latérales proximales 13a, 13b, on peut obtenir un léger serrage de l'avant-bras par effet ressort de cette fourche et, par conséquent, une meilleure tenue du brassard 7 transversalement autour de l'avant-bras. On assure également une pression homogène sur l'avant-bras.

Les deux pattes latérales proximales 13a, 13b du brassard 7 comprennent des moyens d'attache 14 de la sangle 8 constitués ici par des organes faisant saillie vers l'extérieur et pourvus d'une fente 15 longitudinale de passage pour la sangle 8.

L'orthèse peut en outre comporter des organes d'appui 16a, 16b situés à la partie extrême proximale des pattes latérales proximales 13a, 13b, ces organes d'appui 16a, 16b étant tournés vers l'intérieur de l'orthèse 1 en se faisant face. Ces organes d'appui permettent d'assurer un meilleur maintien de l'orthèse sur l'avant-bras de l'utilisateur, sans serrage excessif, ce qui est d'autant plus important que l'orthèse est destinée à être portée de nuit. A cet effet, ils peuvent être réalisés en un matériau anti glisse, tel que le silicone.

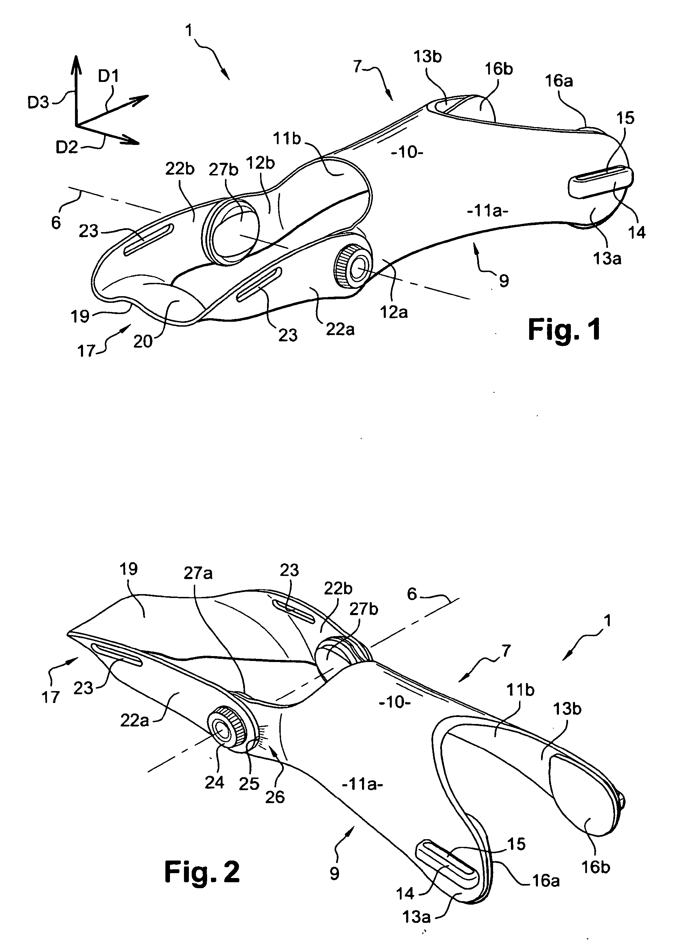

Avantageusement, les organes d'appui 16a, 16b peuvent présenter une forme de canal incurvé selon une direction sensiblement longitudinale, comme on le voit plus particulièrement sur la figure 2. Cette forme en canal épouse la forme de l'avant-bras et améliore encore le maintien.

On décrit à présent la partie distale 4.

La partie distale 4 comporte un support 17 sensiblement rigide, par exemple réalisé en matière plastique ou en résine, destiné à être placé sous la paume de la main et une sangle 18 souple reliée au support 17 et destinée à passer contre le dos de la main (voir figures 6 et 7a à 7e).

Dans la réalisation représentée, le support 17 comporte :
- une portion centrale 19 comportant d'une part un bossage 20 dont la forme est agencée pour épouser la forme de la paume et d'autre part un évidement 21 destiné à recevoir la commissure pouce - index et à permettre le passage du pouce sous le support 17 ;
- deux parois latérales 22a, 22b s'étendant depuis la portion centrale 19 sensiblement longitudinalement dans le sens proximal, de part et d'autre de la main, l'axe de pivotement 6 étant situé sensiblement à la partie extrême proximale desdites parois latérales 22a, 22b.

Les deux parois latérales 22a, 22b du support 17 comprennent des moyens d'attache 23 de la sangle 18 constitués ici par des orifices de passage pour la sangle 18, orientés sensiblement longitudinalement.

Le brassard 7 et le support 17 sont associés l'un à l'autre par des moyens de pivotement autour de l'axe 6 prédéfini. En position assemblée, la partie extrême proximale de chaque paroi latérale 22a, 22b du support 17 est en contact, transversalement, avec la partie extrême distale de la patte latérale distale 12a, 12b correspondante du brassard 7. Au voisinage de l'axe de pivotement 6, les parois latérales 22a, 22b sont ainsi superposées aux pattes latérales distales 12a, 12b, en étant situées à l'extérieur de celles-ci.

L'orthèse 1 comprend également des moyens de limitation de l'amplitude de pivotement du support 17 par rapport au brassard 7, dans une plage angulaire s'étendant de part et d'autre de la position neutre. Par exemple, le pivotement relatif s'effectue dans une plage de ± 20° autour de cette position neutre. Un angle θ positif entre le support 17 et le brassard 7 correspond à une position de la main en extension, tandis qu'un angle θ négatif correspond à une position de la main en flexion.

Avantageusement, l'orthèse comprend en outre des moyens de réglage de l'angle θ entre le support 17 et le brassard 7 selon des valeurs d'angle discrètes, par exemple de 5° en 5°, ainsi que des moyens de blocage des moyens de pivotement dans une position angulaire souhaitée.

Selon une réalisation possible, la liaison entre le brassard 7 et le support 17 est obtenue au moyen d'une denture de petite dimensions (non visible sur les figures) et d'un écrou 24 accessible depuis l'extérieur et formant organe de réglage et de serrage pour l'utilisateur. Afin de simplifier le réglage de l'angle θ, il peut être prévu sur la ou chaque paroi latérale 22a, 22b du support 17 un ergot 25 proximal disposé en regard d'une graduation angulaire 26 réalisée sur la patte latérale distale 12a, 12b correspondante du brassard 7.

De préférence, l'orthèse 1 comprend des organes d'appui 27a, 27b situés au niveau de l'axe de pivotement 6 et tournés vers l'intérieur de l'orthèse 1 en se faisant face. Ces organes d'appui permettent d'assurer un meilleur maintien de l'orthèse sur l'avant-bras de l'utilisateur, sans serrage excessif, ce qui est particulièrement important dans le cas présent, où l'orthèse est destinée à un port nocturne. A cet effet, les organes d'appui peuvent être réalisés en un matériau anti glisse, tel que le silicone. De plus, ils permettent de protéger le poignet de l'utilisateur des moyens de pivotement.

Avantageusement, les organes d'appui 27a, 27b peuvent présenter une forme de canal incurvé selon une direction sensiblement longitudinale, comme on le voit plus particulièrement sur la figure 1. Cette forme en canal épouse la forme du poignet et améliore encore le maintien.

Les figures 6 et 7a à 7e montrent l'orthèse 1 portée par un utilisateur.

L'avant-bras 3 est enserré entre le brassard 7, dont la portion centrale 10 est placée contre la face postérieure de l'avant-bras, et la sangle 8, passant contre la face antérieure de l'avant-bras. La main 5 est enserrée entre le support 17, placé contre la paume, et la sangle 18, passant contre le dos de la main, le pouce passant sous le support 17. Grâce à cette disposition, on obtient un effet bras de levier qui garantit un excellent maintien de l'orthèse 1.

Les figures 7a à 7e montrent différentes positions angulaires relatives entre le support 17 et le brassard 7.

Sur la figure 7c, l'orthèse est dans la position neutre, l'angle θ entre le support 17 et le brassard 7 étant nul. Dans cette position, le poignet du porteur de l'orthèse n'est ni en flexion ni en extension. Sur les figures 7a et 7b, la main est en extension, tandis que sur les figures 7d et 7e, la main est en flexion.

L'orthèse 1 est destiné à être portée la nuit par un utilisateur, qui ne peut alors pas fléchir la main au-delà de l'angle θ. L'angle θ est réglé par l'utilisateur en fonction des indications de son médecin. Par exemple, on peut fixer initialement un angle θ nul, et passer à un angle θ de quelques degrés en extension si les douleurs s'intensifient. A l'inverse, on peut passer à un angle θ de quelques degrés en flexion si les douleurs s'atténuent et augmenter progressivement l'amplitude de flexion autorisée.

L'orthèse 1 est particulièrement simple à régler par l'utilisateur lui-même. Elle ne requiert pas l'intervention d'un orthésiste qualifié pour sa réalisation sur mesure. Il suffit de prévoir une gamme limitée d'orthèses pour le poignet gauche et droit, dans un certain nombre de tailles.

Il va de soi que l'invention n'est pas limitée au mode de réalisation décrit ci-dessus à titre d'exemple mais qu'elle en embrasse au contraire toutes les variantes de réalisation.

## Revendications

1. Orthèse de poignet, destinée au traitement du syndrome du canal carpien, comprenant :
- une partie proximale (7, 8) destinée à enserrer l'avant-bras (3) la partie proximale comportant un brassard (7) sensiblement rigide destiné à être placé contre la face postérieure de l'avant-bras et une sangle (8) souple reliée au brassard (7) et destinée à passer contre la face antérieure de l'avant-bras, de sorte que l'avant-bras soit enserré entre le brassard (7) net la sangle (8);
- une partie distale (17, 18) destinée à enserrer la main (5) ;
- des moyens de pivotement des parties proximale et distale l'une par rapport à l'autre, autour d'un axe de pivotement (6) transversal qui, lorsque l'orthèse (1) est portée par un utilisateur, est sensiblement confondu avec l'axe d'articulation en flexion / extension du poignet de l'utilisateur ;
- des moyens de blocage des moyens de pivotement dans une position angulaire (θ) souhaitée ;
**caractérisée en ce que** sangle (8) souple reliée au brassard (7) et destinée à passer contre la face
la partie distale comporte un support (17) sensiblement rigide destiné à être placé sous la paume de la main et une sangle (18) souple reliée au support (17) et destinée à passer contre le dos de la main, de sorte que la main soit enserrée entre le support (17) et la sangle (18).

2. Orthèse selon la revendication 1, **caractérisée en ce qu'**elle comprend des moyens de limitation de l'amplitude de pivotement de la partie distale (17) par rapport à la partie proximale (7) dans une plage angulaire s'étendant de part et d'autre de la position neutre.

3. Orthèse selon la revendication 1 ou 2, **caractérisée en ce qu'**elle comprend des moyens de réglage de l'angle (θ) entre la partie distale (17) et la partie proximale (7) selon des valeurs d'angle discrètes.

4. Orthèse selon l'une des revendications 1 à 3, **caractérisée en ce que** le brassard (7) comporte :
- une partie principale (9) en forme de gouttière qui, lors du port de l'orthèse (1) par un utilisateur, vient coiffer l'avant-bras, ladite partie principale (9) comprenant une portion centrale (10) placée contre la face postérieure de l'avant-bras et deux portions latérales (11 a, 11 b) disposées de part et d'autre de l'avant-bras;
- deux pattes latérales distales (12a, 12b) s'étendant sensiblement longitudinalement dans le sens distal chacune depuis une portion latérale (11a, 11b) de la partie principale (9), de part et d'autre de l'avant-bras, l'axe de pivotement (6) étant situé sensiblement à la partie extrême distale desdites pattes latérales distales (12a, 12b).

5. Orthèse selon la revendication 4, **caractérisée en ce que** le brassard (7) comporte en outre deux pattes latérales proximales (13a, 13b) s'étendant sensiblement longitudinalement dans le sens proximal chacune depuis une portion latérale (11 a, 11 b) de la partie principale (9), de part et d'autre de l'avant-bras, lesdites pattes latérales proximales (13a, 13b) formant une fourche pouvant être légèrement déformée élastiquement.

6. Orthèse selon la revendication 5, **caractérisée en ce qu'**elle comprend des organes d'appui (16a, 16b) situés à la partie extrême proximale des pattes latérales proximales (13a, 13b), tournés vers l'intérieur de l'orthèse (1) en se faisant face.

7. Orthèse selon l'une des revendications 1 à 6, **caractérisée en ce qu'**elle comprend des organes d'appui (27a, 27b) situés au niveau de l'axe de pivotement (6) et tournés vers l'intérieur de l'orthèse (1) en se faisant face.

8. Orthèse selon la revendication 6 ou 7, **caractérisée en ce que** les organes d'appui (16a, 16b, 27a, 27b) présentent une forme de canal incurvé selon une direction sensiblement longitudinale.

9. Orthèse selon l'une des revendications 1 à 8, **caractérisée en ce que** le support (17) comporte :
- une portion centrale (19) comportant d'une part un bossage (20) dont la forme est agencée pour épouser la forme de la paume et d'autre part un évidement (21) destiné à recevoir la commissure pouce - index et à permettre le passage du pouce sous le support (17) ;
- deux parois latérales (22a, 22b) s'étendant depuis la portion centrale (19) sensiblement longitudinalement dans le sens proximal, de part et d'autre de la main, l'axe de pivotement (6) étant situé sensiblement à la partie extrême proximale desdites parois latérales (22a, 22b).

## Claims

1. A wrist orthosis intended for the treatment of carpal tunnel syndrome, comprising:
- a proximal part (7, 8) intended to be fitted around the forearm (3), the proximal part comprising a substantially rigid armband (7) intended to be positioned against the posterior face of the forearm, and flexible a strap (8) connected to the armband (7) and intended to be passed over the anterior face of the forearm, so that the forearm is gripped between the armband (7) and the strap (8);
- a distal part (17, 18) intended to be fitted around the hand (5);
- pivot means for pivoting of the proximal and distal parts relative to one another around a transverse pivot axis (6) which, when the orthosis (1) is worn by a user, substantially merges with the flexion/extension articulating axis of the user's wrist;
- means for blocking the pivot means in a desired angle position (θ);
**characterized in that** the distal part comprises a substantially rigid support (17) intended to be placed under the palm of the hand, a flexible strap (18) connected to the support (17) and intended to be passed over the back of the hand, so that the hand is gripped between the support (17) and the strap (18).

2. The orthosis according to claim 1, **characterized in that** it comprises means for limiting the pivoting amplitude of the distal part (17) relative to the proximal part (7) over an angle range extending on either side of the neutral position.

3. The orthosis according to claim 1 or 2, **characterized in that** it comprises means for adjusting the angle (θ) between the distal part (17) and the proximal part (7) according to discrete angle values.

4. The orthosis according to one of claims 1 to 3, **characterized in that** the armband (7) comprises:
- a main part (9) in the form of a cradle which, when the orthosis (1) is worn by the user, is placed over the forearm, said main part (9) comprising a central portion (10) positioned against the posterior face of the forearm and two side portions (11a, 11b) arranged on either side of the forearm;
- two distal side extensions (12a, 12b) extending substantially longitudinally in the distal direction, each from a side portion (11a, 11 b) of the main part (9), on either side of the forearm, the pivot axis (6) being situated substantially at the distal end part of said distal side extensions (12a, 12b).

5. The orthosis according to claim 4, **characterized in that** the armband (7) further comprises two proximal side extensions (13a, 13b) extending substantially longitudinally in the proximal direction each from a side portion (11 a, 11 b) of the main part (9) on either side of the forearm, said proximal side extensions (13a, 13b) forming a fork which can be slightly deformed elastically.

6. The orthosis according to claim 5, **characterized in that** it comprises bearing members (16a, 16b) located at the proximal end part of the proximal side extensions (13a, 13b) facing inwardly towards the orthosis (1) and facing one another.

7. The orthosis according to one of claims 1 to 6, **characterized in that** it comprises bearing members (27a, 27b) located at the pivot axis (6) and facing inwardly towards the orthosis (1) facing one another.

8. The orthosis according to claim 6 or 7 **characterized in that** the bearing members (16a, 16b, 27a, 27b) have a channel shape curved in a substantially longitudinal direction.

9. The orthosis according to one of claims 1 to 8, **characterized in that** the support (17) comprises:
- a central portion (19) comprising, on the one hand, a raised part (20) whose shape is arranged to follow the shape of the palm and, on the other hand, a recess (21) intended to receive the thumb-index commissure and to allow the passing of the thumb underneath the support (17);
- two side walls (22a, 22b) extending from the central portion (19) substantially longitudinally in the proximal direction, on either side of the hand, the pivot axis (6) being situated substantially at the proximal end part of said side walls (22a, 22b).

## Patentansprüche

1. Handgelenkorthese zur Behandlung des Karpaltunnelsyndroms, die umfasst:
- einen proximalen Abschnitt (7, 8), der dazu bestimmt ist, den Unterarm (3) zu umschließen, wobei der proximale Abschnitt eine etwa starre Armbinde (7) aufweist, die dazu bestimmt ist, an der Hinterseite des Unterarms platziert zu sein und einen elastischen Gurt (8), der mit der Armbinde (7) verbunden ist und dazu bestimmt, an der Vorderseite des Unterarms entlang geführt zu sein, so dass der Unterarm zwischen der Armbinde (7) und dem Gurt (8) umschlossen ist,
- einen distalen Abschnitt (17, 18), der dazu bestimmt ist, die Hand (5) zu umschließen,
- Schwenkmittel des proximalen und distalen Abschnitts zueinander um eine transversale Schwenkachse (6), die, wenn die Orthese (1) von einem Benutzer getragen wird, etwa mit der Gelenkachse für die Beugung/Streckung des Handgelenks des Benutzers zusammenfällt,
- Blockiermittel der Schwenkmittel in einer gewünschten Winkelposition (θ),
**dadurch gekennzeichnet, dass**
der distale Abschnitt etwa starre eine Unterlage (17) aufweist, die dazu bestimmt ist, unter der Handfläche der Hand platziert zu sein und einen elastischen Gurt (18), der mit der Unterlage (17) verbunden ist und dazu bestimmt, am Handrücken entlang geführt zu sein, so dass die Hand zwischen der Unterlage (17) und dem Gurt (18) umschlossen ist.

2. Orthese nach Anspruch 1, **dadurch gekennzeichnet, dass** sie Mittel zur Begrenzung der Schwenkamplitude des distalen Abschnitts (17) im Verhältnis zum proximalen Abschnitt (7) in einem Winkelbereich hat, der sich auf der einen und der anderen Seite der neutralen Stellung erstreckt.

3. Orthese nach Anspruch 1 oder 2, **dadurch gekennzeichnet, dass** sie Einstellmittel des Winkels (θ) zwischen dem distalen Abschnitt (17) und dem proximalen Abschnitt (7) gemäß diskreten Winkelwerten umfasst.

4. Orthese nach einem der Ansprüche 1 bis 3, **dadurch gekennzeichnet, dass** die Armbinde (7) umfasst:
- einen Hauptabschnitt (9) in Rinnenform, der beim Tragen der Orthese (1) durch einen Benutzer den Unterarm bedeckt, wobei der Hauptabschnitt (9) einen zentralen Teil (10) umfasst, der an der Hinterseite des Unterarms platziert ist und zwei Seitenteile (11a, 11b), die auf der einen und der anderen Seite des Unterarms angeordnet sind,
- zwei distale seitliche Zungen (12a, 12b), die sich jeweils von einem Seitenteil (11 a, 11 b) des Hauptabschnitts (9) auf der einen und der anderen Seite des Unterarms etwa längs in die distale Richtung erstrecken, wobei sich die Schwenkachse (6) etwa am äußersten distalen Abschnitt der distalen seitlichen Zungen (12a, 12b) befindet.

5. Orthese nach Anspruch 4, **dadurch gekennzeichnet, dass** die Armbinde (7) ferner zwei proximale seitliche Zungen (13a, 13b) aufweist, die sich jeweils von einem Seitenteil (11 a, 11 b) des Hauptabschnitts (9) auf der einen und der anderen Seite des Unterarms etwa längs in die proximale Richtung erstrecken, wobei die proximalen seitlichen Zungen (13a, 13b) eine Gabel bilden, die elastisch leicht verformbar ist.

6. Orthese nach Anspruch 5, **dadurch gekennzeichnet, dass** sie Stützorgane (16a, 16b) umfasst, die sich am proximalen Endabschnitt der proximalen seitlichen Zungen (13a, 13b) befinden und zur Innenseite der Orthese (1) zeigen, wobei sie sich gegenüberliegen.

7. Orthese nach einem der Ansprüche 1 bis 6, **dadurch gekennzeichnet, dass** sie Stützorgane (27a, 27b) umfasst, die sich auf Ebene der Schwenkachse (6) befinden und zur Innenseite der Orthese (1) zeigen, wobei sie sich gegenüberliegen.

8. Orthese nach Anspruch 6 oder 7, **dadurch gekennzeichnet, dass** die Stützorgane (16a, 16b, 27a, 27b) eine Form eines gemäß einer etwa länglichen Richtung gekrümmten Kanals aufweisen.

9. Orthese nach einem der Ansprüche 1 bis 8, **dadurch gekennzeichnet, dass** die Unterlage (17) umfasst:
- einen zentralen Teil (19), der auf der einen Seite eine Wulst (20) aufweist, deren Form ausgebildet ist, um die Form der Handfläche anzunehmen und auf der anderen Seite eine Aussparung (21), die dazu bestimmt ist, die Kommissur Daumen-Zeigefinger aufzunehmen und den Durchgang des Daumens unter der Unterlage (17) zu erlauben,
- zwei Seitenwände (22a, 22b), die sich auf der einen und der anderen Seite der Hand ab dem zentralen Teil (19) etwa längs in die proximale Richtung erstrecken, wobei sich die Schwenkachse (6) etwa im proximalen Endabschnitt der Seitenwände (22a, 22b) befindet.
